(19) Europäisches Patentamt
European Patent Office
Office européen des brevets

(11) Veröffentlichungsnummer: **0 037 495**
**A1**

(12) # EUROPÄISCHE PATENTANMELDUNG

(21) Anmeldenummer: 81102088.2

(51) Int. Cl.³: **C 07 D 403/14**

(22) Anmeldetag: 20.03.81

(30) Priorität: 27.03.80 DE 3011809

(43) Veröffentlichungstag der Anmeldung:
14.10.81 Patentblatt 81/41

(84) Benannte Vertragsstaaten:
AT BE CH DE FR GB IT LI NL SE

(71) Anmelder: CASSELLA Aktiengesellschaft
Hanauer Landstrasse 526
D-6000 Frankfurt am Main 61(DE)

(72) Erfinder: Bender, Heinz, Dr.
Kirchgasse 20
D-6000 Frankfurt/Main 60(DE)

(72) Erfinder: Raabe, Thomas, Dr.
Schillerstrasse 5
D-6451 Rodenbach(DE)

(72) Erfinder: Bohn, Helmut, Dr.
Kranzbergring 11
D-6369 Schöneck(DE)

(72) Erfinder: Martorana, Piero, Dr.
Kaiser-Friedrich-Promenade 108
D-6380 Bad Homburg(DE)

(74) Vertreter: Urbach, Hans-Georg, Dr. et al,
Hanauer Landstrasse 526
D-6000 Frankfurt am Main 61(DE)

(54) **Pyrimidyl-chinazoline, Verfahren zu ihrer Herstellung, sie enthaltende pharmazeutische Präparate und ihre Verwendung.**

(57) Pyrimidyl-chinazoline der Formel I

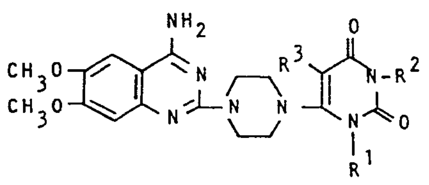

(I)

worin R¹ und R² Wasserstoff, Alkyl mit 1 bis 6 C Atomen, Alkenyl mit 2 bis 6 C Atomen, Cycloalkyl mit 5 bis 7 C Atomen, R³ Wasserstoff, Alkyl mit 1 bis 6 C Atomen, Alkenyl mit 2 bis 6 C Atomen, Phenyl, Phenalkyl mit 1 bis 4 C Atomen im Alkylrest, Halogen, Nitro, Amino, Alkylcarbonylamino mit 1 bis 6 C-Atomen im Alkylrest, Phenylcarbonylamino oder Formyl bedeuten, werden dadurch hergestellt, daß a) eine Verbindung der Formel II

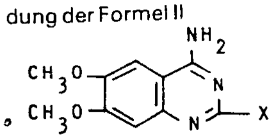

(II)

wobei X einen Mercaptorest oder ein Halogenatom bedeutet, mit einer Verbindung der Formel III

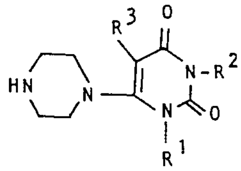

(III)

oder b) das 2-Piperazinyl-4-amino-6,7-dimethoxy-chinazolin mit einer Verbindung der Formel V

./...

EP 0 037 495 A1

Croydon Printing Company Ltd

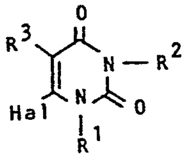

(V)

worin Hal ein Halogenatom bedeutet, umgesetzt wird, oder c) in eine Verbindung der Formel VI

Ref. 3181 A
Dr.Eu/Hz/Ll/St

# Pyrimidyl-chinazoline, Verfahren zu ihrer Herstellung, sie enthaltende pharmazeutische Präparate und ihre Verwendung

Die Erfindung betrifft pharmakologisch wertvolle Pyrimidyl-chinazoline der Formel I

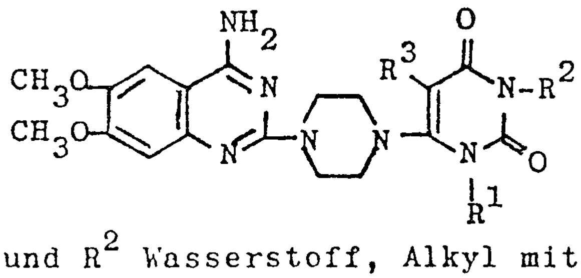

(I)

worin $R^1$ und $R^2$ Wasserstoff, Alkyl mit 1 bis 6 C Atomen, Alkenyl mit 2 bis 6 C Atomen, Cycloalkyl mit 5 bis 7 C Atomen, $R^3$ Wasserstoff, Alkyl mit 1 bis 6 C Atomen, Alkenyl mit 2 bis 6 C Atomen, Phenyl, Phenalkyl mit 1 bis 4 C Atomen im Alkylrest, Halogen, Nitro, Amino, Alkylcarbonylamino mit 1 bis 6 C-Atomen im Alkylrest, Phenylcarbonylamino oder Formyl bedeuten, sowie die Säureadditionssalze der Verbindungen I, ihre Herstellung und Verwendung.

Verbindungen der Formel I, bei denen $R^1 = R^2$ ist, sind, ebenso wie die Säureadditionssalze dieser Verbindungen, besonders bevorzugt. Die für $R^1$, $R^2$ und $R^3$ stehenden Alkyl- und Alkenylgruppen können geradkettig oder verzweigt sein. Bei dem für $R^1$ und $R^2$ stehenden Cycloalkylrest ist Cyclopentyl und Cyclohexyl bevorzugt. Bei dem Phenalkylrest ist Benzyl und Phenethyl bevorzugt. Halogen bedeutet insbesondere Chlor, Fluor oder Brom. Bei dem Alkylcarbonylaminorest ist Acetylamino bevorzugt.

Beispiele für geeignete Alkyl- und Alkenylreste sind: Methyl, Ethyl, n-Propyl, i-Propyl, n-Butyl, i-Butyl, sec-Butyl, tert-Butyl, n-Pentyl, i-Pentyl, n-Hexyl, Allyl, Methallyl. Für $R^3$ sind Methyl, Ethyl, n-Propyl, n-Butyl, Brom oder Amino bevorzugt. Ganz besonders bevorzugt sind Verbindungen der Formel I

bzw. deren Säureadditionssalze, bei denen $R^1 = R^2$ = Methyl und $R^3$ = Wasserstoff oder Nitro bedeuten.

Die Verbindungen der Formel I können, sofern mindestens einer der Substituenten $R^1$, $R^2$ oder $R^3$ Wasserstoff bedeutet, auch in anderen tautomeren Formen vorkommen.

Zur Herstellung der Verbindungen der Formel I kann man eine Verbindung der Formel II

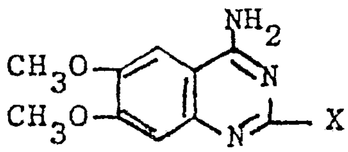

(II)

wobei in Formel II X einen Mercaptorest oder insbesondere ein Halogenatom, vorzugsweise Chlor oder Brom bedeutet, mit einer Verbindung der Formel III

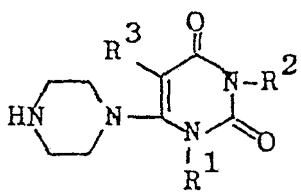

(III)

wobei in Formel III $R^1$, $R^2$ und $R^3$ die bereits angegebene Bedeutung besitzen, umsetzen. Diese Umsetzung wird normalerweise in einem geeigneten Lösungs- oder Dispergiermittel durchgeführt, in dem die Reaktionspartner gelöst bzw. suspendiert werden, wie z.B. einem Kohlenwasserstoff, beispielsweise Benzol, Toluol, Xylol, einem halogenierten Kohlenwaaserstoff, wie z.B. Chloroform, Methylenchlorid, Tetrachlorkohlenstoff, Chlorbenzol, einem Ether, wie z.B. Dioxan, Diethylether, Tetrahydrofuran, ferner Wasser, Dimethylsulfoxyd, Dimethylformamid, N-Methylpyrrolidon, einem Alkohol, wie z.B. Methanol, Ethanol, Amylalkohol, Isoamylalkohol usw. Die Reaktion kann bei Normaltemperatur oder erhöhter Temperatur , z.B. 20 bis 160°C durchgeführt werden . Die Reaktionstemperatur beträgt normalerweise 80 bis 140°C. Bei einem niedrigeren Siedepunkt des verwendeten Lösungs- oder Dispergiermittels wird die Reaktion in einem geschlossenen Druckgefäß durchgeführt. Das Molverhältnis zwischen den Verbindungen der Formeln II und III beträgt im allgemeinen 1 : 1. Setzt man gleichmolare Mengen ein, so ist

es bei Verwendung einer Verbindung II mit X = Halogen empfehlenswert, in Gegenwart von mindestens äquimolaren Mengen eines säurebindenden Mittels, wie z.B. Pottasche, Soda, Triethylamin usw. zu arbeiten. Ohne säurebindende Mittel erhält man sonst gewöhnlich die Hydrohalogenide der Verbindungen der allgemeinen Formel I. Setzt man eine der Verbindungen II oder III im molaren Überschuß ein, so kann der Überschuß der Verbindung als säurebindendes Mittel wirken.

Die Herstellung der Ausgangsverbindung der Formel II kann nach bekannten Vorschriften (z.B. Soc. 1948, S. 1764 oder US-PS 3 511 836) erfolgen.

Die Ausgangsverbindung der Formel II mit X = Cl ist bekannt, vgl. DE-OS 1 620 138, Spalte 7 und US-PS 3 511 836, Beispiel 1. Die Ausgangsverbindungen der Formel II, bei denen X ein anderes Halogenatom als Chlor bedeutet, können entsprechend hergestellt werden. So kann z.B. das bekannte 2,4-Dihydroxy-6,7-dimethoxychinazolin mit Phosphoroxybromid in das 2,4-Dibrom-6,7-dimethoxychinazolin und dieses mit wasserfreiem Ammoniak in das 2-Brom-4-amino-6,7-dimethoxychinazolin überführt werden. Die Ausgangsverbindung der Formel II mit $X = -SCH_3$ ist ebenfalls bekannt, vgl. DE-OS 1 620 138, Spalte 7 - 8. Die Ausgangsverbindungen der Formel III können durch Umsetzung der bekannten oder nach an sich bekannten Methoden herstellbaren Pyrimidinen der Formel V

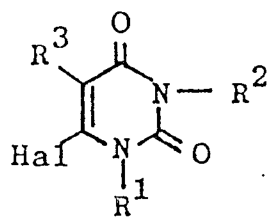

(V)

worin Hal ein Halogenatom, vorzugsweise Chlor bedeutet, mit Piperazin erhalten werden. Die Umsetzung eines Pyrimidins der Formel V mit Piperazin wird normalerweise in einem geeigneten Lösungs- oder Dispergiermittel durchgeführt, in dem die Reaktionspartner gelöst bzw. suspendiert werden, wie z.B.

einem Kohlenwasserstoff, beispielsweise Benzol, Toluol, Xylol, einem halogenierten Kohlenwasserstoff wie z.B. Chloroform, Methylenchlorid, Tetrachlorkohlenstoff, Chlorbenzol, einem Ether, wie z.B. Dioxan, Diethylether, Tetrahydrofuran, ferner Wasser, Dimethylsulfoxyd, Dimethylformamid, N-Methylpyrrolidon, einem Alkohol wie z.B. Methanol, Ethanol, Amylalkohol, Isoamylalkohol usw. Die Reaktion kann bei Normaltemperatur oder erhöhter Temperatur z.B. 20 bis 160°C ausgeführt werden. Normalerweise beträgt die Reaktionstemperatur 80 bis 120°C Bei einem niedrigeren Siedepunkt des verwendeten Lösungs- oder Dispergiermittels wird die Reaktion in einem geschlossenen Druckgefäß durchgeführt. Das Molverhältnis zwischen dem Pyrimidin der Formel V und Piperazin kann 1:(1 bis 10) und gegebenenfalls noch mehr betragen. Setzt man gleichmolare Mengen ein, so ist es empfehlenswert, in Gegenwart von mindestens äquimolaren Mengen eines säurebindenden Mittels, wie z.B. Pottasche, Soda, Triethylamin usw. zu arbeiten. Ohne säurebindende Mittel erhält man gewöhnlich die Hydrohalogenide der Verbindungen der Formel III.

Zur Herstellung von Verbindungen der Formel I kann man auch das 2-Piperazinyl-4-amino-6,7-dimethoxychinazolin der Formel IV

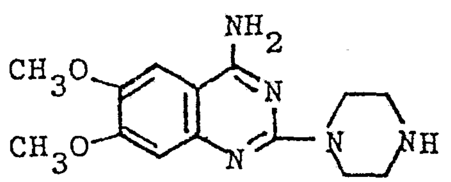

(IV)

mit einer Verbindung der Formel V umsetzen. Die Umsetzung des Chinazolinderivats der Formel IV mit einem Pyrimidin der Formel V wird normalerweise in einem geeigneten Lösungs- oder Dispergiermittel durchgeführt, in der die Reaktionspartner gelöst bzw. suspendiert werden, wie z.B. einem Kohlenwasserstoff, beispielsweise Benzol, Toluol, Xylol, einem halogenierten Kohlenwasserstoff, wie z.B. Chloroform, Methylenchlorid, Tetrachlorkohlenstoff, Chlorbenzol, einem Ether,

wie z.B. Dioxan, Diethylether, Tetrahydrofuran, ferner Wasser, Dimethylsulfoxyd, Dimethylformamid, N-Methylpyrrolidon, einem Alkohol, wie z.B. Methanol, Ethanol, Amylalkohol, Isoamylalkohol usw. Die Reaktion kann bei Normaltemperatur oder erhöhter Temperatur, z.B. 20 bis 160°C durchgeführt werden. Normalerweise beträgt die Reaktionstemperatur 80 bis 140°C Bei einem niedriegeren Siedepunkt des verwendeten Lösungs- oder Dispergiermittels wird die Reaktion in einem geschlossenen Druckgefäß durchgeführt. Das Molverhältnis zwischen beiden Verbindungen beträgt im allgemeinen 1:1. Setzt man gleichmolare Mengen ein, so ist es empfehlenswert, in Gegenwart von mindestens äquimolaren Mengen eines säurebindenden Mittels, wie z.B. Pottasche, Soda, Triethylamin usw. zu arbeiten. Ohne säurebindende Mittel erhält man sonst gewöhnlich die Hydrohalogenide der Verbindungen der Formel I. Setzt man einen molaren Überschuß der Formel IV ein, so kann dieser Überschuß als säurebindendes Mittel wirken.

Das Chinazolin der Formel IV kann nach bekannten Vorschriften (z.B. DE-OS 1 620 138) hergestellt werden. Ein weiteres Herstellungsverfahren für die erfindungsgemäßen Verbindungen der Formel I, für die $R^3$ ungleich Wasserstoff ist, besteht darin, daß man von den in 5-Stellung des Pyrimidinrings unsubstituierten Verbindungen der Formel VI

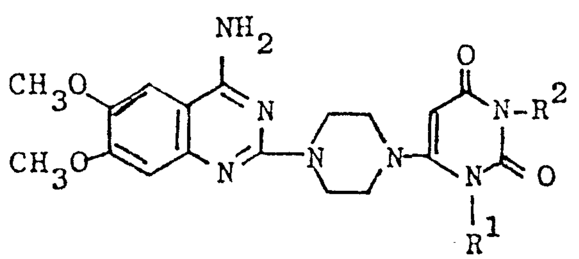

(VI)

ausgeht und in an sich bekannter Weise die Substituenten $R^3$ in die 5-Stellung einführt, so z.B. die Formylgruppe durch Umsetzung von VI mit Essigsäure/Ameisensäureanhydrid, die Nitrogruppe durch vorsichtige Nitrierung mit Salpetersäure, die Amino- und Alkylcarbonyl- oder Phenylcarbonylaminogruppe durch Reduktion der Nitrogruppe und gegebenenfalls Acylierung

mit Acylierungsmitteln, welche die Alkylcarbonyl- bzw. Phenylcarbonylgruppe einführen, den Bromrest durch vorsichtige Bromierung usw.

Zur Bildung von Säureadditionssalzen mit den Verbindungen der Formel I sind anorganische und organische Säuren geeignet, beispielsweise Chlorwasserstoff, Bromwasserstoff, Naphthalindisulfonsäure(1,5) , Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-Isoicotin- , Methansulfon-, p-Toluolsulfon-, Citronen- oder Adipinsäure. Pharmazeutisch annehmbare Säureadditionssalze werden bevorzugt. Die Säureadditionssalze können wie üblich durch Vereinigung der Komponenten, zweckmäßigerweise in einem geeigneten Verdünnungs- bzw. Dispergiermittel, erhalten werden. Geeignete Verdünnungs- bzw. Dispergiermittel sind z.B. Ether, Wasser, Alkohole, Kohlenwasserstoffe etc.

Die erfindungsgemäßen Verbindungen der Formel I und ihre pharmazeutisch akzeptablen Säureadditionssalze besitzen wertvolle pharmakologische Eigenschaften. Insbesondere besitzen sie stark ausgeprägte blutdrucksenkende Wirkungen und sind daher zur Behandlung von Hypertension am Menschen geeignet. Überraschenderweise sind die Verbindungen der Formel I den bekannten ähnlich gebauten Verbindungen der US-PS 3 511 836 deutlich überlegen. Die erfindungsgemäßen Chinazolylpyrimidine können daher am Menschen für sich allein, in Mischungen untereinander oder in pharmazeutischen Zubereitungen verabreicht werden, die als aktiven Bestandteil eine wirksame Dosis mindestens eines erfindungsgemäßen Chinazolylpyrimidins oder eines Säureadditionssalzes davon neben üblichen pharmazeutisch einwandfreien Träger- und Zusatzstoffen enthalten. Geeignete Trägerstoffe sind z.B. Wasser, pflanzliche Öle, Stärke, Gelatine,

Milchzucker, Magnesiumstearat, Wachse, Vaseline usw. Als Zusatzstoffe können z.B. Netzmittel, Sprengmittel, Konservierungsmittel usw. verwendet werden. Die pharmazeutischen Präparate können in Form von z.B. Tabletten, Kapseln, wäßrigen oder öligen Lösungen oder Suspensionen, Emulsionen, injizierbaren wässrigen oder öligen Lösungen oder Suspensionen, dispergierbaren Pulvern oder Aerosolmischungen vorliegen. Die pharmazeutischen Präparate können neben den Verbindungen der Formel I auch noch eine oder mehrere andere pharmakologisch wirksame Substanzen, beispielsweise Beruhigungsmittel, wie z.B. Luminal, Meprobamat, Chlorpromazine und Benzodiazepinsedativa, wie z.B. Diazepam oder Chlordiazepoxid, Vasodilatoren, wie z.B. Glyzerintrinitrat, Pentaerythrittetranitrat und Carbochromen, das Herz tonisierende Mittel, wie z.B. Digitalis-Präparate, ß-Blocker, wie z.B. Propranolol, Bronchodilatoren und sympathomimetische Mittel, wie z.B. Isoprenalin, Orciprenalin usw., $\alpha$-adrenergische Blockierungsmittel, wie z.B. Phentolamin, Diuretica, wie z.B. Furosemid enthalten.

Die Herstellung der Verbindungen der Formel I wird an folgenden Beispielen erläutert:

<u>Beispiel 1</u>

2,9 g 2-Piperazinyl-4-amino-6,7-dimethoxy-chinazolin werden in 50 ml Ethanol unter Zugabe von 4 g Pottasche und 2,2 g 1,3-Dimethyl-5-nitro-6-chlor-2,4(1H,3H)-pyrimidindion 24 Stunden bei Raumtemperatur gerührt. Dann wird abgesaugt und der Rückstand in 20 ml 0,5 n wäßriger Salzsäure digeriert.

Es wird abermals abgesaugt, der Rückstand in wäßriger Sodalösung suspendiert, abgesaugt und aus wäßrigem Dimethylformamid umkristallisiert.

Man erhält so das 4-(4-Amino-6,7-dimethoxychinazolin-2-yl)-1-(1,2,3,4-tetrahydro-1,3-dimethyl-2,4-dioxo-5-nitro-pyrimidin-6-yl)-piperazin der Formel

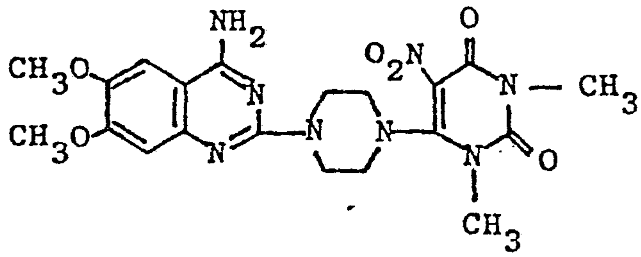

Fp: 300°C

Analyse: ($C_{20}H_{24}N_8O_6$)

| | C | H | N | O |
|---|---|---|---|---|
| berechnet: | 50,8 | 5,1 | 23,7 | 20,3 |
| gefunden: | 50,9 | 5,1 | 23,5 | 20,4 |

Ausbeute: 87 % der Theorie.

Das als Ausgangsprodukt verwendete 1,3-Dimethyl-5-nitro-6-chlor-2,4(1H,3H)-pyrimidindion kann wie folgt hergestellt werden: Zu 50 ml konzentrierte Schwefelsäure gibt man bei 15°C portionsweise 17 g 1,3-Dimethyl-6-chlor-2,4(1H,3H)-pyrimidindion. Dann wird auf 3 bis 5°C abgekühlt und zu der Mischung langsam unter Rühren 17 ml rauchende Salpetersäure zugetropft. Anschließend wird die Lösung unter Rühren auf Eis gegossen, wobei ein halbfester Niederschlag ausfällt. Die Mischung wird mit Methylenchlorid extrahiert, die organische Phase mit Magnesiumsulfat getrocknet und die Lösung im Wasserstrahlvakuum bei 25°C eingeengt. Es hinterbleibt ein Öl, das nach kurzer Zeit fest wird. Die Substanz wird einmal aus Ligroin/Essigester umkristallisiert. Man erhält so das 1,3-Dimethyl-5-nitro-6-chlor-2,4(1H,3H)-pyrimidindion in einer Ausbeute von 95 % der Theorie. Fp: 92°C.

Das als Ausgangsprodukt benutzte 2-Piperazinyl-4-amino-6,7-dimethoxy-chinazolin kann wie folgt hergestellt werden:

Zu einer Lösung von 5 g wasserfreiem Piperazin in 50 ml Dioxan gibt man 2,5 g 2-Chlor-4-amino-6,7-dimethoxychinazolin und erhitzt dann die Mischung 12 Stunden auf 95°C. Dann

wird eingeengt, der Rückstand in Wasser gelöst und mit Salzsäure auf pH 2,5 gestellt. Die saure wäßrige Lösung wird mit Methylenchlorid extrahiert, dann die wäßrige Phase mit Natronlauge alkalisch gestellt. Es fällt einNiederschlag aus, der aus Ethanol umkristallisiert wird. Man erhält so das 2-Piperazinyl-4-amino-6,7-dimethoxy-chinazolin in einer Ausbeute von 83 % der Theorie. Fp: 235°C.

Beispiel 2

2,4g 2-Chlor-4-amino-6,7-dimethoxy-chinazolin werden zusammen mit 4.5 g 1,3-Dimethyl-6-piperazinyl-2,4(1H,3H)-pyrimidindion zusammen mit 50 ml Ethanol im Autoklaven 8 Stunden auf 130°C erhitzt. Dann wird die Mischung abgekühlt und im Wasserstrahlvakuum eingeengt. Der Rückstand wird mit Wasser digeriert und anschließend aus Dimethylformamid umkristallisiert. Man erhält so das 4-(4-Amino-6,7-dimethoxy-chinazolin-2-yl)-1-(1,2,3,4-tetrahydro-1,3-dimethyl-2,4-dioxo-pyrimidin-6-yl)-piperazin

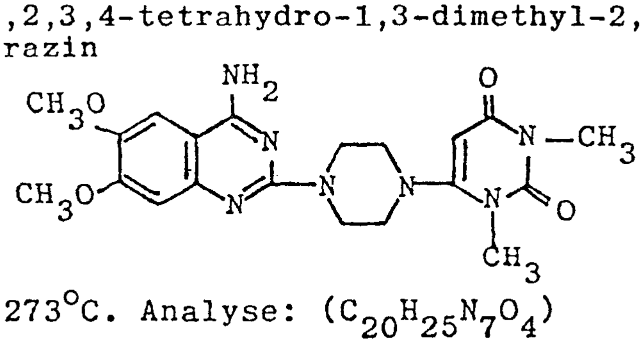

Fp: 273°C. Analyse: ($C_{20}H_{25}N_7O_4$)

| | C | H | N | O |
|---|---|---|---|---|
| berechnet: | 56,2 | 5,9 | 23,0 | 15,0 |
| gefunden: | 56,0 | 6,0 | 23,0 | 14,7 |

Ausbeute: 81 % der Theorie.

Das als Ausgangsprodukt benutzte 1,3-Dimethyl-6-piperazinyl-2,4(1H,3H)-pyrimidindion kann wie folgt hergestellt werden: Zu einer Mischung von 30 g Piperazin in 500 ml Toluol gibt man 20 g 1,3-Dimethyl-6-chlor-2,4(1H,3H)-pyrimidindion und kocht die Mischung 3 Stunden am Rückfluß. Dann wird abgesaugt und das Filtrat im Wasserstrahlvakuum eingeengt. Der nach dem Einengen zurückbleibende Rückstand wird aus Essigester

umkristallisiert. Man erhält so das 1.3-Dimethyl-6-piperazinyl-2,4(1H,3H)-pyrimidindion

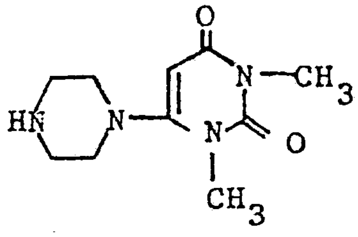

in einer Ausbeute von 75 % der Theorie. Fp: 117°C.

Beispiel 3

5,2g 4-(4-Amino-6,7-dimethoxychinazolin-2-yl)-1-(1,2,3,4-tetrahydro-1,3-dibutyl-2,4-dioxo-pyrimidin-6-yl)-piperazin

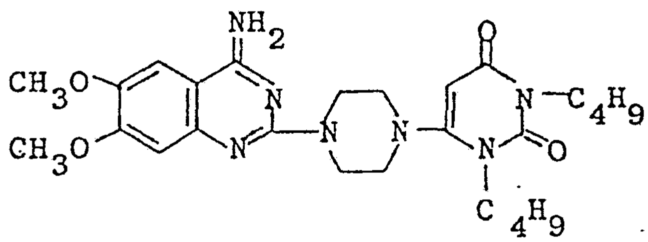

werden in 20 ml Eisessig gelöst. Dann tropft man unter Kühlen und Eiskühlung eine Mischung von 1,7 g Brom in 20 ml Eisessig zu. Anschließend läßt man 2 Stunden bei Raumtemperatur nachrühren. Dann wird abgesaugt und aus Ethanol umkristallisiert. Man erhält so das 1-(4-Amino-6,7-dimethoxychinazolin-2-yl)-1-(1,2,3,4-tetrahydro-1,3-dibutyl-2,4-dioxo-5-brom-pyrimidin-6-yl)-piperazin-hydrobromid

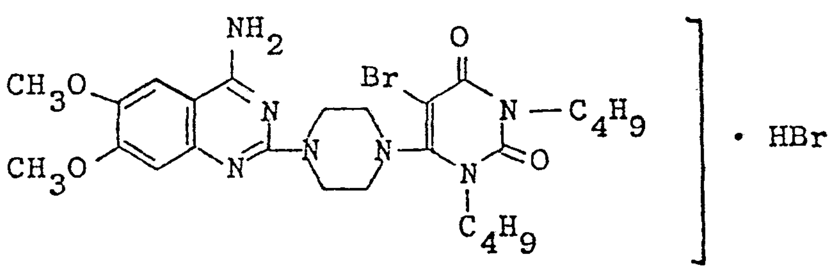

Fp: über 300°C Zers. Analyse: ($C_{26}H_{37}Br_2N_7O_4$)

| | C | H | Br | N | O |
|---|---|---|---|---|---|
| berechnet: | 46,5 | 5,5 | 23,8 | 14,6 | 9,5 |
| gefunden: | 46,7 | 5,3 | 23,5 | 14,7 | 9,8 |

Ausbeute: 61 % der Theorie

Beispiel 4

Zu einem Gemisch aus 24 ml Acetanhydrid und 12 ml Ameisensäure werden 5,6 g 4-(4-Amino-6,7-dimethoxychinazolin-2-yl)-1(1,2,3,4-tetrahydro-1,3-dihexyl-2,4-dioxo-pyrimidin-6-yl)-piperazin

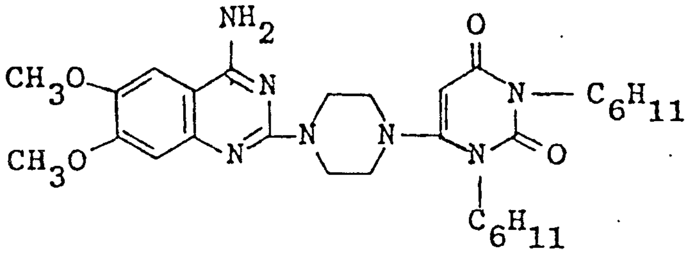

zugesetzt und die Mischung 5 Stunden bei 80°C gerührt. Anschließend wird im Wasserstrahlvakuum eingeengt. Der Rückstand wird mit 0,5 n wäßriger Natronlauge durchgearbeitet, abgesaugt und aus Dimethylformamid umkristallisiert. Man erhält so 4-(4-Amino-6,7-dimethoxychinazolin-2-yl)-1-(1,2,3,4-tetrahydro-1,3-dihexyl-2,4-dioxo-5-formyl-pyrimidin-6-yl)-piperazin

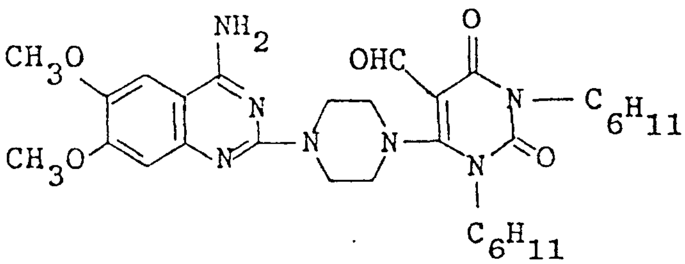

Fp: 314°C Zers. Analyse: ($C_{31}H_{41}N_7O_5$)

| | C | H | N | O |
|---|---|---|---|---|
| berechnet: | 62,9 | 6,9 | 16,6 | 13,5 |
| gefunden: | 62,8 | 6,6 | 16,5 | 13,2 |

Ausbeute: 81 % der Theorie

Beispiel 5

4,7 g 4-(4-Amino-6,7-dimethoxychinazolin-2-yl)-1-(1,2,3,4-tetrahydro-1,3-dimethyl-2,4-dioxo-5-nitro-pyrimidin-6-yl)-piperazin

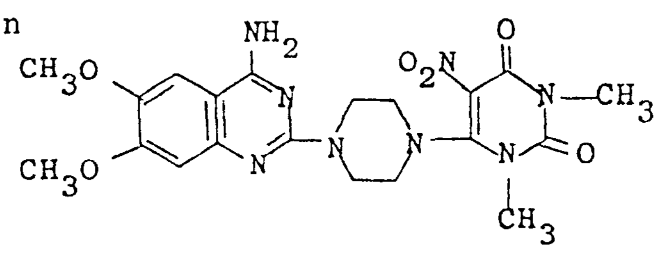

werden in 300 ml Ethanol suspendiert, 0,4 g Pd/Kohle

(10%ig) zugesetzt und die Mischung 20 Stunden bei Raumtemperatur und Normaldruck unter Schütteln mit Wasserstoff hydriert. Danach wird abgesaugt, der Rückstand sofort mit Dimethylformamid heiß digeriert und die Dimethylformamidlösung noch heiß vom Katalysator abfiltriert. Anschließend wird die Dimethylformamidlösung im Wasserstrahlvakuum eingeengt und der Rückstand aus wäßrigem Dimethylformamid umkristallisiert. Man erhält so das 4-(4-Amino-6,7-dimethoxychinazolin-2-yl)-1-(1,2,3,4-tetrahydro-1,3-dimethyl-2,4-dioxo-5-amino-pyrimidin-6-yl)-piperazin

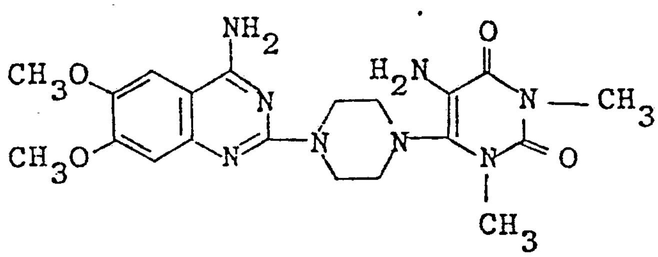

Fp: 327°C. Analyse ($C_{20}H_{26}N_7O_4$)

| | C | H | N | O |
|---|---|---|---|---|
| berechnet: | 54,3 | 5,9 | 25,3 | 14,5 |
| gefunden: | 54,0 | 6,1 | 25,0 | 14,7 |

Ausbeute: 78 % der Theorie.

Analog den Beispielen 1 bis 5 wurden folgende Substanzen hergestellt:

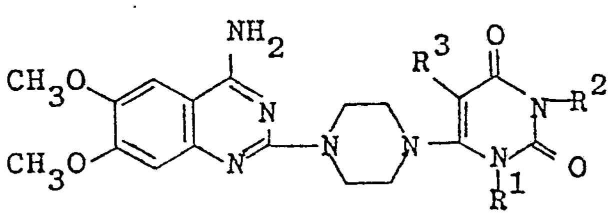

| $R^1 = R^2$ | $R^3$ | Fp: |
|---|---|---|
| $-CH_3$ | $-CH_3$ | 317°C |
| 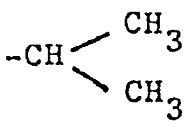 | $-C_4H_9$ | 325°C |
| H | H | 297°C |
| $-CH_3$ | $-NH-COOC_2H_5$ | 314°C |
| $-C_3H_7$ | $-CH_2-$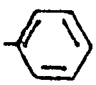 | 331°C |
| $CH_2=CH-CH_2-$ | 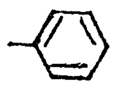 | 319°C |
| H | $-C_6H_{11}$ | 322°C |
| $-CH_3$ | $-CH_2-CH=CH_2$ | 328°C |
| 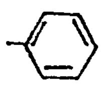 | H | 316°C |
| $-CH_2-$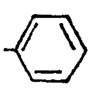 | H | 324°C |
| $-C_2H_5$ | H | 311°C |
| $-C_4H_9$ | H | 309°C |
| $-C_6H_{11}$ | H | 323°C |
| $-C_2H_5$ | $NO_2$ | 318°C |
| $-C_3H_7$ | CHO | 331°C |
| $-C_3H_7$ | H | 314°C |

| $R^1$ | $R^2$ | $R^3$ | Fp: |
|---|---|---|---|
| $CH_3$ | $C_2H_5$ | H | 303°C |
| H | $C_2H_5$ | H | 298°C |

Die pharmakologische Wirksamkeit der erfindungsgemäßen Verbindungen wurde wie folgt geprüft:

1. Blutdruckmessung an der normotonen narkotisierten Ratte

Männliche IVANOVAS-Ratten (300 - 340 g) des Stammes SIV 50 wurden durch Applikation von 66 mg/kg α-Chloralose (1,1 % Lösung, 0,6 ml/100 g) und 20 mg:kg Aprobarbital in die Schwanzvene narkotisiert. Die Trachea wurde kannuliert um die Spontanatmung zu erleichtern. Die Messung des Blutdruckes erfolgte blutig über einen in die Rechte A. carotis eingebundenen PE-Schlauch auf konventionelle Weise. Mittels eines Dreiwegehahnes konnte dieser PE-Schlauch zur i.a.-Applikation der Prüfsubstanzen benutzt werden, wobei jeweils ein Volumen von 0,1 ml injiziert wurde. Durch Nachspülen mit 0,9 % NaCl gelangten die Substanzen über die Vertebralarterie auch in Richtung Zentralnervensystem bevor die Verteilung im allgemeinen Kreislauf erfolgte.

Zahlenmäßig ausgewertet wurde die maximale systolische und diastolische Blutdrucksenkung sowie die maximale Änderung der Herzfrequenz. Als Wirkdauer galt die Zeit vom Eintritt der Veränderung bis zum Erreichen des Ausgangswertes. Es wurden die in der nachfolgenden Tabelle 1 angegebenen Werte erhalten:

Tab.1

| Präparat und Dosis (mg/kg) | $BD_s$ Δ mmHg $\bar{x}$ | WD min $\bar{x}$ | $BD_d$ Δ mmHg $\bar{x}$ | HF Δ s/min $\bar{x}$ |
|---|---|---|---|---|
| * 0.01 | - 30 | 36 | - 27 | + 5 |
| Prazosin 0.1 | - 20 | 60 | - 17 | 0 |

$BD_s$ = Blutdruck systolisch

$BD_d$ = Blutdruck diastolisch

WD = Wirkungsdauer

HF = Herzfrequenz

$\bar{x}$ = Mittelwert

* = 4-(4-Amino-6,7-dimethoxychinazolin-2-yl)-1-(1,2,3,4-tetrahydro-1,3-dimethyl-2,4-dioxo-5-nitro-pyrimidin-6-yl)-piperazin

Prazosin = Vergleichspräparat gemäß US Patentschrift 3511836

2. Blutdruckwirkung am narkotisierten normotonen Hund

Die Untersuchungen wurden an Bastardhunden beiderlei Geschlechts in Pentobarbital-Narkose (30 - 40 mg/kg i.v. ) durchgeführt. Die Beatmung der Tiere erfolgte mit einem Bird-Mark-7-Respirator. Der endexpiratorische Kohlensäuregehalt (gemessen mit dem Uras) betrug zwischen 4,5 und 5 Vol-%.

Während des gesamten Versuches erhielten die Tiere eine Dauerinfusion von Pentobarbital i.v.: 4 mg/kg/6 ml/h, um eine konstante Narkosetiefe zu gewährleisten.

Der systolische und diastolische Blutdruck wurde peripher in der A. femoralis über einen Statham-Druckaufnehmer gemessen.

Die Parameter wurden kontinuierlich auf einem Brush-Mark-6-Direktschreiber über zugehörige Vorverstärker registriert.

Die Prüfpräparate wurden i.v. als Bolus injiziert. Es wurden die in der nachfolgenden Tabelle 2 angegebenen Werte erhalten:

Tab. 2

| Präparat und Dosis (mg/kg) | $BD_s$ mmHg $\bar{x}$ | WD min $\bar{x}$ | $BD_d$ mmHg $\bar{x}$ | HF s/min $\bar{x}$ |
|---|---|---|---|---|
| * 0.1 | - 40 | 60 | - 42 | - 17 |
| Prazosin 0.5 | - 15 | 10 | - 10 | 0 |

$BD_s$ = Blutdruck systolisch
$BD_d$ = Blutdruck diastolisch
WD = Wirkungsdauer
HF = Herzfrequenz
$\bar{x}$ = Mittelwert
* = 4-(4-Amino-6,7-dimethoxychinazolin-2-yl)-1-(1,2,3,4-tetrahydro-1,3-dimethyl-2,4-dioxo-5-nitro-pyrimidin-6-yl)-piperazin
Prazosin = Vergleichspräparat gemäß US Patentschrift 3511836

Im Falle oraler Applikation wird im allgemeinen pro menschliches Individuum eine tägliche Dosis von etwa 0,1 bis 100 mg, vorzugsweise 0,5 bis 40 mg, der aktiven Substanz verabreicht. Auch bei anderen Applikationsformen liegt die Dosis in ähnlichen Bereichen, das heißt, im allgemeinen ebenso bei 0,1 bis 100 mg pro menschliches Individuum.
Die tägliche Dosis wird normalerweise in mehrere, beispielsweise 2 bis 4, Einzeldosen aufgeteilt, wobei die einzelne Dosis 0,001 bis 3 mg pro kg Körpergewicht von der aktiven Substanz enthält. Die Konzentration der aktiven Substanz pro Einzeldosis in den Zubereitungen ist 0,01 bis 20 %, vorzugsweise 0,05 bis 10 %, bezogen auf das Gesamtgewicht der Zubereitung.

Beispiel 6

Bei der Herstellung von Pillen kann folgende Rezeptur verwendet werden:

| | |
|---|---|
| 4-(4-Amino-6,7-dimethoxychinazolin-2-yl)-1-(1,2,3,4-tetrahydro-1,3-dimethyl-2,4-dioxo-5-nitro-pyrimidin-6-yl-) piperazin | 2 mg |
| Kornstärke | 100 mg |
| Lactose | 60 mg |
| Sek. Calciumphosphat | 30 mg |
| Lösliche Stärke | 2 mg |
| Magnesiumstearat | 2 mg |
| Kolloidale Kieselsäure | 4 mg |
| | 200 mg |

Beispiel 7

Tabletten können nach folgender Rezeptur hergestellt werden:

| | |
|---|---|
| 4-(4-Amino-6,7-dimethoxy-chinazolin-2-yl)-1-(1,2,3,4-tetrahydro-1,3-dimethyl-2,4-dioxo-pyrimidin-6-yl)-piperazin | 2 mg |
| Lactose | 60 mg |
| Kornstärke | 30 mg |
| Lösliche Stärke | 4 mg |
| Magnesiumstearat | 4 mg |
| | 100 mg |

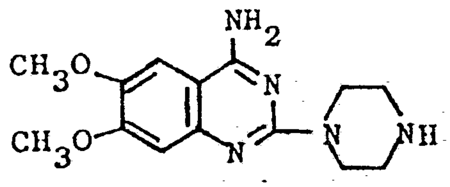

(IV)

der Substituent $R^3$ in 5-Stellung des Pyrimidindion-Kerns eingeführt wird.

PATENTANSPRÜCHE

1. Pyrimidyl-chinazolin der Formel I

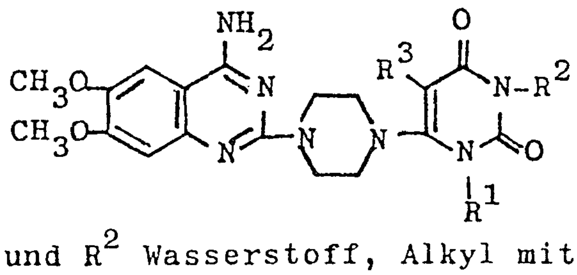

(I)

worin $R^1$ und $R^2$ Wasserstoff, Alkyl mit 1 bis 6 C Atomen, Alkenyl mit 2 bis 6 C Atomen, Cycloalkyl mit 5 bis 7 C Atomen, $R^3$ Wasserstoff, Alkyl mit 1 bis 6 C Atomen, Alkenyl mit 2 bis 6 C Atomen, Phenyl, Phenalkyl mit 1 bis 4 C Atomen im Alkylrest, Halogen, Nitro, Amino, Alkylcarbonylamino mit 1 bis 6 C-Atomen im Alkylrest, Phenylcarbonylamino oder Formyl bedeuten, sowie die Säureadditionssalze der Verbindungen I.

2.Pyrimidyl-chinazolin nach Anspruch 1, dadurch gekennzeichnet, daß $R^1 = R^2$ ist.

3. Pyrimidyl-chinazolin nach Anspruch 1, dadurch gekennzeichnet, daß $R^1 = R^2$ = Methyl ist.

4. Pyrimidyl-chinazolin nach den Ansprüchen 1 bis 3, dadurch gekennzeichnet, daß $R^3$ Wasserstoff bedeutet.

5. Pyrimidyl-chinazolin nach den Ansprüchen 1 bis 3, dadurch gekennzeichnet, daß $R^3$ Nitro bedeutet.

6. Verfahren zur Herstellung von Pyrimidyl-chinazolinen der Formel I gemäß den Ansprüchen 1 bis 5, dadurch gekennzeichnet, daß a) eine Verbindung der Formel II

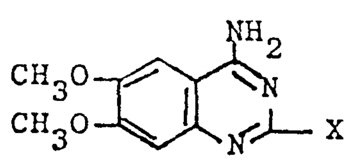

(II)

wobei X einen Merkaptorest oder insbesondere ein Halogenatom, vorzugsweise Chlor oder Brom bedeutet, mit einer Verbindung der Formel III

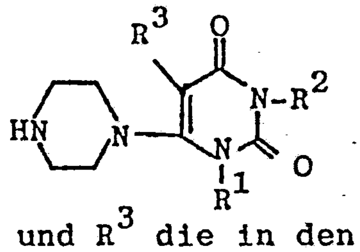

(III)

wobei $R^1$, $R^2$ und $R^3$ die in den Ansprüchen 1 bis 5 angegebenen Bedeutungen besitzen, oder b) das 2-Piperazinyl-4-amino-6,7-dimethoxy-chinazolin mit einer Verbindung der Formel V

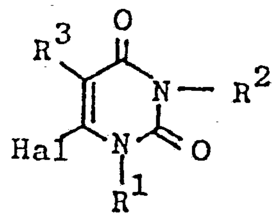

(V)

worin Hal ein Halogenatom, vorzugsweise Chlor, bedeutet und wobei $R^1$, $R^2$ und $R^3$ die in den Ansprüchen 1 bis 5 angegebenen Bedeutungen besitzen, umgesetzt wird, oder c) in eine Verbindung der Formel VI

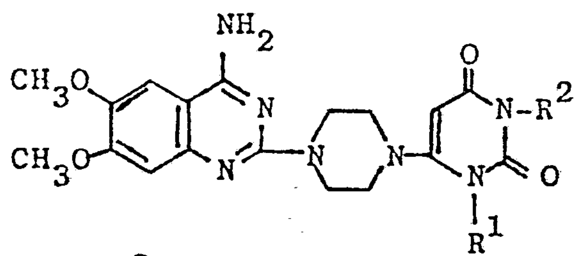

(VI)

der Substituent $R^3$ in 5-Stellung des 2,4(1H,3H)-Pyrimidindion-Kerns eingeführt wird, wobei $R^1$, $R^2$ und $R^3$ die in den Ansprüchen 1 bis 5 angegebenen Bedeutungen besitzen, und die erhaltene Verbindung der Formel I gegebenenfalls in ein Säureadditionssalz überführt wird.

7. Pharmazeutische Zubereitung, enthaltend eine wirksame Dosis eines Pyrimidyl-chinazolins der allgemeinen Formel I oder einem Säureadditionssalz davon, nach einem der Ansprüche 1 bis 5 neben pharmazeutisch zulässigen Träger- und Zusatzstoffen, gegebenenfalls zusätzlich enthaltend noch eine oder mehrere andere pharmazeutisch wirksame Substanzen, insbesondere ausgewählt aus β-Blockern, Sedativa, Vasodilatoren, Diuretica,

Kardialmembranstabilisierungsmitteln, kardiotonischen Mitteln, α-adrenergischen Blockierungsmitteln und sympathomimetischen Mitteln.

8. 4-(4-Amino-6,7-dimethoxy-chinazolin-2-yl)-1-(1,2,3,4-tetrahydro-1,3-dimethyl-2,4-dioxy-5-nitro-pyrimidin-6-yl)-piperazin und seine Säureadditionssalze.

9. 4-(4-Amino-6,7-dimethoxy-chinazolin-2-yl)-1-(1,2,3,4-tetrahydro-1,3-dimethyl-2,4-dioxy-pyrimidin-6-yl)-piperazin und seine Säureadditionssalze.

10. Verwendung der Pyrimidyl-chinazoline der Ansprüche 1 bis 5, 8 oder 9 oder deren Säureadditionssalze zur Behandlung von Kreislauf- oder Herzbeschwerden.

Patentanspruchsfassung für Österreich

1. Verfahren zur Herstellung von Pyrimidyl-chinazolinen der Formel I

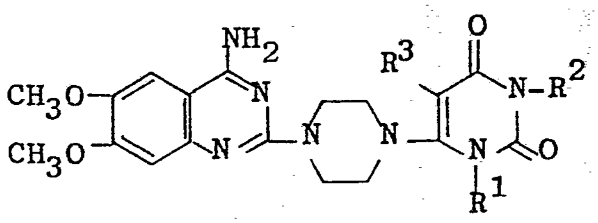

(I)

worin $R^1$ und $R^2$ Wasserstoff, Alkyl mit 1 bis 6 C Atomen, Alkenyl mit 2 bis 6 C Atomen, Cycloalkyl mit 5 bis 7 C Atomen, $R^3$ Wasserstoff, Alkyl mit 1 bis 6 C Atomen, Alkenyl mit 2 bis 6 C Atomen, Phenyl, Phenalkyl mit 1 bis 4 C Atomen im Alkylrest, Halogen, Nitro, Amino, Alkylcarbonylamino mit 1 bis 6 C Atomen im Alkylrest, Phenylcarbonylamino oder Formyl bedeuten, sowie ihre Säureadditionssalze, dadurch gekennzeichnet, daß a) eine Verbindung der Formel II

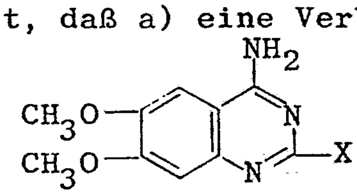

(II)

wobei X einen Merkaptorest oder insbesondere ein Halogenatom, vorzugsweise Chlor oder Brom, bedeutet, mit einer Verbindung der Formel III

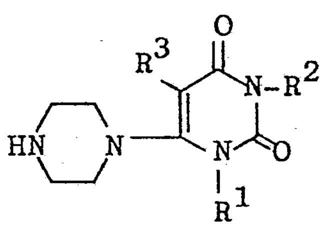

(III)

oder b) daß 2-Piperazinyl-4-amino-6,7-dimethoxy-chinazolin mit einer Verbindung der Formel V

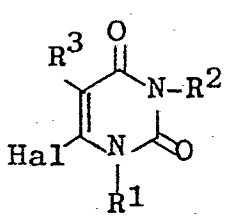

(V)

worin Hal ein Halogenatom, vorzugsweise Chlor, bedeutet, umgesetzt wird oder c) in eine Verbindung der Formel VI

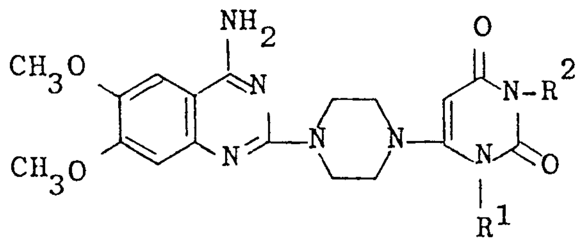

(VI)

der Substituent $R^3$ in 5-Stellung des 2,4(1H,3H)-Pyrimidindion-Kerns eingeführt wird und die erhaltene Verbindung der Formel I gegebenenfalls in ein Säureadditionssalz überführt wird.

2. Verfahren nach Anspruch 1, dadurch gekennzeichnet, daß die Ausgangsverbindungen so ausgewählt werden, daß $R^1 = R^2$ ist.

3. Verfahren nach Anspruch 1, dadurch gekennzeichnet, daß die Ausgangsverbindungen so ausgewählt werden, daß $R^1 = R^2 =$ Methyl ist.

4. Verfahren nach den Ansprüchen 1 bis 3, dadurch gekennzeichnet, daß die Ausgangsverbindungen so ausgewählt werden, daß $R^3$ Wasserstoff bedeutet.

5. Verfahren nach den Ansprüchen 1 bis 3, dadurch gekennzeichnet, daß die Ausgangsverbindungen so ausgewählt werden, daß $R^3$ Nitro bedeutet.

6. Verwendung der nach den Verfahren der Ansprüche 1 bis 5 hergestellten Pyrimidylchinazoline oder ihrer Säureadditionssalze zur Herstellung von Pharmazeutika, insbesondere von Kreislaufmitteln und Antihypertensiva, auf nicht chemischem Wege.

7. Verwendung der Pyrimidyl-chinazoline der Formel I

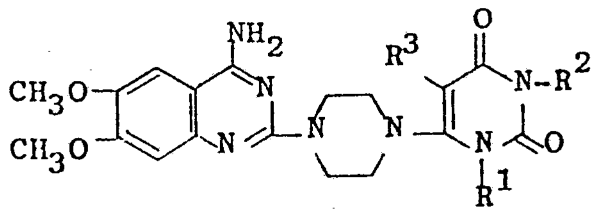

(I)

worin $R^1$ und $R^2$ Wasserstoff, Alkyl mit 1 bis 6 C Atomen, Alkenyl mit 2 bis 6 C Atomen, Cycloalkyl mit 5 bis 7 C Atomen, $R^3$ Wasserstoff, Alkyl mit 1 bis 6 C Atomen, Alkenyl mit 2 bis 6 C Atomen, Phenyl, Phenalkyl mit 1 bis 4 C Atomen im Alkylrest, Halogen, Nitro, Amino, Alkylcarbonylamino mit 1 bis 6 C Atomen im Alkylrest, Phenylcarbonylamino oder Formyl bedeuten, sowie ihre Säureadditionssalze zur Behandlung von Kreislauf- oder Herzbeschwerden.

8. Verfahren nach den Ansprüchen 1 bis 3 und 4, dadurch gekennzeichnet, daß die Ausgangsverbindungen so ausgewählt werden, daß das 4-(4-Amino-6,7-dimethoxy-chinazolin 2-yl)-1-(1,2,3,4-tetrahydro-1,3-dimethyl-2,4-dioxy-5-nitro-pyrimidin-6-yl)-piperazin und seine Säureadditionssalze entsteht.

9. Verfahren nach den Ansprüchen 1 bis 3, dadurch gekennzeichnet, daß die Ausgangsverbindungen so ausgewählt werden, daß das 4-(4-Amino-6,7-dimethoxy-chinazolin-2-yl)-1-(1,2,3,4-tetrahydro-1,3-dimethyl-2,4-dioxy-pyrimidin-6-yl)-piperazin und seine Säureadditionssalze entsteht.

0037495

Europäisches Patentamt

**EUROPÄISCHER RECHERCHENBERICHT**

Nummer der Anmeldung
EP 81 10 2088

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich, der maßgeblichen Teile | betrifft Anspruch | |
| A | GB - A - 2 007 656 (PFIZER)<br>* Seiten 1-4, 6-12, 18-20 *<br>--------- | 1,6,7 | C 07 D 403/14 |
| | | | RECHERCHIERTE SACHGEBIETE (Int Cl ) |
| | | | C 07 D 403/14 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt | | |

| Recherchenort | Abschlußdatum der Recherche | Prüfer |
|---|---|---|
| Den Haag | 18.07.1981 | FRANCOIS |

EPA form 1503.1 06 78